# EUROPÄISCHE PATENTSCHRIFT

(11) **EP 1 151 991 B1**
(45) Veröffentlichungstag und Bekanntmachung des Hinweises auf die Patenterteilung: **23.07.2003**
(21) Anmeldenummer: 01109429.9
(22) Anmeldetag: 23.04.2001
(51) Int. Cl.: C07C 263/16

(54) **Verfahren zur Herstellung organischer Monoisocyanate**
Process for the production of organic monoisocyanates
Procédé pour la production de monoisocyanates organiques

(30) Priorität: 04.05.2000 DE 10021742
(43) Veröffentlichungstag der Anmeldung: 07.11.2001
(73) Patentinhaber: BAYER AG, 51368 Leverkusen (DE)
(72) Erfinder: Brahm, Dr.Martin, 51519 Odenthal (DE); Schmalstieg, Dr.Lutz, 50676 Köln (DE); Geron, Ulrich, 56637 Plaidt (DE); Behr, Hans-Günter, 42799 Leichlingen (DE); Morawski, Jörg, 42799 Leichlingen (DE)

(56) Entgegenhaltungen:
- EP-A- 0 307 756
- EP-A- 0 341 515

## Beschreibung

Die Erfindung betrifft ein neues phosgenfreies Verfahren zur Herstellung organischer Monoisocyanate durch thermische Zersetzung von in situ hergestellten Biuret/Harnstoffgruppen enthaltenden Polyisocyanaten.

Die Herstellung von organischen Isocyanatverbindungen mit Phosgen ist seit langem bekannt und in einer Vielzahl von Veröffentlichungen und Patenten beschrieben (z.B. Houben-Weyl, Methoden der organischen Chemie Band 8, S. 120ff (Georg Thieme Verlag Stuttgart 1952)). Eine sichere Handhabung von Phosgen setzt eine spezielle Technik und aufwendige Herstellungs- und Sicherheitssysteme voraus. Daher hat es nicht an Versuchen gefehlt, durch phosgenfreie Methoden Isocyanatverbindungen zu synthetisieren.

Eine an sich bekannte und einfache Labormethode zur phosgenfreien Herstellung von Isocyanaten ist eine thermische Zersetzung von Biuret- bzw. Harnstoffstrukturen aufweisenden Verbindungen. Hierbei wird zu einem Überschuss an hochsiedender Isocyanatverbindung ein niedermolekulares Amin oder Polyamin zudosiert und bei Temperaturen von 200 °C und mehr das in einer Gleichgewichtsreaktion entstandene niedrigsiedende Isocyanat abdestilliert. In der Veröffentlichung Bunge W. Angew. Chem. 72, 1002 (1960) wird auf diese "Einfache Laboratoriumsmethode zur Herstellung niedersiedender Isocyanate" eingegangen. Auch in der Veröffentlichung Siefken, Annalen der Chemie 562, 81 (1949) wird diese Methode skizziert, wobei Temperaturen oberhalb 200 °C beschrieben werden. In der Patentanmeldung EP-A 307 756 und in der Veröffentlichung W. Mormann, G. Leukel, Synthesis 12, 990f (1988) ist dieses Verfahrensprinzip für spezielle Siloxyisocyanate aufgegriffen und optimiert worden.

Eine Optimierung der genannten Verfahren ist aus einer Vielzahl von Gründen schwierig.

So reagieren bei Temperaturen von 200 °C und mehr Polyisocyanate nicht mehr kontrollierbar und zum Teil unter Gasentwicklung zu hochmolekularen Folgeprodukten, so dass ein technisches Verfahren bei derartig hohen Temperaturen nicht vorhersehbare Reaktionsverläufe annehmen kann.

Eine Austragung der hochmolekularen Folgeprodukte aus dem Reaktionsreaktor ist aufgrund einer zu hohen Viskosität im technischen Maßstab kaum möglich. Ein äquimolarer Umsatz von hochsiedendem Polyisocyanat mit dem niedersiedendem Amin, wie z.B. in der EP-A 307 756 beschrieben, ist aus Viskositätsgründen technisch nicht möglich.

Es war daher Aufgabe der vorliegenden Erfindung, ein breit anwendbares, einfaches und phosgenfreies Verfahren zur Herstellung von Monoisocyanaten zur Verfügung zu stellen, welches besonders in großtechnischem Maßstab gut durchführbar ist.

Diese Aufgabe konnte mit dem nachstehend näher beschriebenen erfindungsgemäßen Verfahren gelöst werden.

Gegenstand der Erfindung ist ein Verfahren zur Herstellung von niedrigsiedenden Monoisocyanaten mit einem Siedepunkt bei Normaldruck zwischen 70 und 320°C
A) aus einer hochsiedenden Isocyanatkomponente durch Umsetzung mit
B) einem Monoamin mit einer primären Aminogruppe in situ zu den entsprechenden Biuret/Harnstoffgruppen-haltigen Isocyanaten und deren gleichzeitiger Spaltung,
C) Abdestillieren des gebildeten Monoisocyanats gegebenenfalls unter Vakuum,
dadurch gekennzeichnet, dass
als hochsiedende Isocyanatkomponente Isocyanatverbindungen mit einem Siedepunkt bei Normaldruck von mindestens 180°C und einem HC-Gehalt von mindestens 50 ppm eingesetzt werden, die Reaktion bei einer maximalen Reaktionstemperatur von 180°C in einem Verhältnis von Isocyanatgruppen zu Aminogruppen von mindestens 4 durchgeführt wird.

Als hochsiedende Isocyanatverbindungen A) sind alle Isocyanatgruppen aufweisenden Verbindungen und Gemische zu verstehen, deren Siedepunkte bei Normalbedingungen über 180 °C, vorzugsweise über 250 °C und besonders bevorzugt über 300 °C liegen.

Die Destillationstemperatur dieser Isocyanatkomponente A) muss bei den Reaktionsbedingungen mindestens 10 °C, vorzugsweise 20 °C besonders bevorzugt 40 °C über der eingestellten Reaktionstemperatur liegen.

Als Isocyanatkomponente A) beispielhaft genannt seien an sich bekannte
Monoisocyanate mit aliphatisch, cycloaliphatisch, araliphatisch oder aromatisch gebundenen Isocyanatgruppen wie z. B. Stearylisocyanat, Naphthylisocyanat,
Diisocyanate mit aliphatisch, cycloaliphatisch, araliphatisch und/oder aromatisch gebundenen Isocyanatgruppen, wie z. B. 1,4-Diisocyanatobutan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 1-Isocyanato-1-methyl-4(3)isocyanatomethylcyclohexan (IMCI), Bis-(isocyanatomethyl)-norboman, 2,4- und 2,6-Diisocyanatotoluol (TDI), 2,4'- und 4,4'-Diisocyanatodiphenylmethan und höhere Homolage, 1,5-Diisocyanatonaphthalin, Dipropylenglycoldiisocyanat,
Triisocyanate und/oder höherfunktioneller Isocyanate wie z. B. 4-Isocyanatomethyl-1,8-octandiisocyanat (Nonantriisocyanat), 1,6,11-Undecantriisocyanat oder beliebige Gemische solcher Isocyanatverbindungen.

Ebenfalls einsetzbar sind sogenannte modifizierte Isocyanatverbindungen die sich aus den oben erwähnten Diisocyanaten und Triisocyanaten ableiten und durch Oligomerisierungsreaktionen wie beispielsweise Trimerisation hergestellt werden.

Es können auch beliebige Mischungen der genannten Isocyanate eingesetzt werden.

Vorzugsweise werden Isocyanatverbindungen eingesetzt, die aromatisch gebundene Isocyanatgruppen enthalten. Besonders bevorzugt werden als Isocyanatkomponente A) Polyisocyanate der Diphenylmethanreihe mit einem Zweikerngehalt (Summe 2,2-, 2,4- und 4,4-Diphenylmethandiisocyanat) von mindestens 85 Gew.-% bezogen auf Gesamtgewicht der Isocyanatkomponente A) eingesetzt.

Es ist erfindungswesentlich, dass die Isocyanatkomponente A) einen HC-Gehalt (Gehalt an hydrolisierbaren Chlorverbindungen) von mindestens 50 ppm, vorzugsweise mindestens 150 ppm und besonders bevorzugt mindestes 300 ppm aufweist. Dies kann sichergestellt werden, indem entweder die Isocyanatkomponente A) bereits herstellungsbedingt einen ausreichenden hohen Chlorgehalt aufweist oder durch Zugabe von Verbindungen, die hydrolisierbares Chlor enthalten. Beispiele für derartige Verbindungen sind Benzoylchlorid, Terephthaloyldichlorid und Isophthaloyldichlorid.

Die Bestimmung des hydrolisierbaren Chlorgehalts in der Isocyanatkomponente A) erfolgt nach an sich bekannten Methoden.

Als niedermolekulare Monoamine B) können beliebige aliphatische, cycloaliphatische oder aromatische Verbindungen eingesetzt werden, die eine primäre Aminogruppe aufweisen und deren als Reaktionsprodukte entstehenden Monoisocyanate bei Reaktionsbedingungen durch Destillation aus dem Reaktionsgemisch entfernt werden können. Die Monoamine können neben der Aminogruppe zusätzlich andere funktionelle Gruppen enthalten, die bei den Reaktionsbedingungen inert gegenüber Isocyanatgruppen sind. Die Monoamine sind direkt ohne spezielle Reinigung in der technisch verfügbaren Reinheit einsetzbar.

Als Monoamine beispielhaft genannt seien C₃-C₁₈-Alkylamine wie die isomeren Butylamine, Pentylamine, Hexylamine, Heptylamine, Octylamine, Nonylamine, Decylamine, Dodecylamin, C₃₋₁₈-Alkylenamine wie Allylamin, und die auf ungesättigten, gegebenenfalls langkettigen Fettsäuren basierenden Monoamine, C₅-C₁₈-Cycloalkylamine wie Cyclohexylamin und aromatische Amine wie Phenylamin, o/p-Fluorphenylamin, o/p Chlorphenylamin, Naphthylamin sowie Alkylphenylamine undHalogenatome enthaltende Alkylphenylamine. Die Kohlenstoffketten der Amine können durch Sauerstoff- und/oder Schwefelatome unterbrochen sein.

Bevorzugt werden Monoamine eingesetzt, die eine aromatisch gebundene Aminogruppe enthalten. Besonders bevorzugt werden halogenhaltige Aniline eingesetzt.

Die erfindungsgemäß hergestellten Monoisocyante C) leiten sich aus den eingesetzten Monoaminen B) ab und müssen bei den vorgegebenen Reaktionsbedingungen destillierbar sein. Sie weisen bei Normaldruck einen Siedepunkt von mindestens 70°C, vorzugsweise mindestens 110°C und höchstens 320°C vorzugsweise höchstens 240°C auf. Im allgemeinen liegt das Molekulargewicht derartiger Monoisocyanate zwischen 83 und 270 g/mol.

In dem erfindungsgemäßen Verfahren wird die hochsiedende Isocyanatkomponente A) in einem molaren Verhältnis von mindestens 4 : 1, vorzugsweise 5 : 1 bis 20 : 1, besonders bevorzugt 6 :1 bis 8 : 1 der Isocyanatgruppen zu primären Aminogruppen bei einer Temperatur von unter 180°C, vorzugsweise 80°C bis 160°C, besonders bevorzugt 120°C bis 140°C vorgelegt und anschließend mit der Monoaminkomponente B) versetzt. Die Monoaminkomponente B) kann hierbei als Reinstoff oder in Abmischung mit anderen nichtreaktiven Verbindungen zudosiert werden. Vorzugsweise wird das Monoamin gelöst in einem bei den Verfahrensbedingungen nicht siedenden Lösungsmittel zudosiert, wobei vorzugsweise Konzentrationen von 10 bis 90 %, besonders bevorzugt 40 - 60 % Verwendung finden. Beispielhaft als Lösungsmittel genannt seinen Verbindungen wie hochsiedende Trialkyl- oder Tritoluylphosphate.

Die Reaktions- und Sumpftemperatur im Reaktionsreaktor ist auf maximal 180°C begrenzt. Vorzugsweise wird bei Reaktionstemperaturen von 100°C bis 170°C und besonders bevorzugt von 120°C bis 160°C gearbeitet.

Die Entfernung des hergestellten Monoisocyanats c) durch Destillation kann drucklos oder unter vermindertem Druck erfolgen. Vorzugsweise wird unter einem verminderten Druck destilliert, wobei besonders bevorzugt bei einem Druck von 5 bis 200 mbar destilliert wird.

Das erfindungsgemäße Verfahren erlaubt eine technisch einfache Herstellung von Monoisocyanaten in Ausbeuten von über 70 % bei gleichzeitiger guter Handhabung des angefallenen Sumpfprodukts.

So hergestellte Monoisocyanate weisen im allgemeinen eine Reinheit von über 90 %, vorzugsweise über 99 % auf und können somit direkt ohne weitere Aufarbeitung in Modifizierungsreaktionen und als Zwischenprodukt weiter eingesetzt werden.

### Beispiele

Die Bestimmung des hydrolysierbaren Chlorgehaltes erfolgte mittels potentiometrischer Titration:

Die zu analysierende Probe wird mit Methanol versetzt und 10 Minuten unter Rückfluss urethanisiert. Anschließend wird die erhaltene Mischung nach Verdünnen mit Wasser durch Kochen unter Rückfluss hydrolisiert. Das hierbei gebildete ionogene Chlor wird nach Ansäuern mit Salpetersäure und Aufstocken mit einer bekannten Masse Natriumchlorid argentometrisch mit einer Silbernitratmaßlösung titriert. Die Titration wird mit inkrementeller Reagenzdosierung und automatischer Equivalenzpunkt-Auswertung driftkontrolliert (Gleichgewichtstitration) durchgeführt.

### Beispiel 1 (erfindungsgemäß)

In einen Reaktionsreaktor mit Destillationseinheit werden bei Raumtemperatur 420 kg eines technischen Polyisocyanats der Diphenylmethanreihe mit einem 2 Kern-Anteil von 90Gew% (Desmodur® MDI 90/10, Handelsprodukt der Bayer AG, NCO-Gehalt: 32 %, Viskosität: 13 mPas, HC-Gehalt: 357 ppm, bestimmt nach potentiometrischer Methode) unter Vakuum eingezogen und nach Belüftung mit Stickstoff auf 130 °C aufgeheizt.

Bei dieser Temperatur werden 53,7 kg n-Hexylamin aus einer Vorlage so in den Reaktor eindosiert, dass die Reaktorinnentemperatur nicht über 150°C ansteigt (deutlich exotherme Reaktion, Kühlung erforderlich).

Nach beendeter Hexylamin-Zugabe wird die Kesselinnentemperatur auf 160°C eingestellt und vorsichtig evakuiert, bis ein deutlicher Rückfluss zu erkennen ist. Danach wird der Rücklaufteiler der Destillationsanlage auf 5 Teile Abnahme und 1 Teil Rücklauf eingestellt. Hierbei destilliert das entstehende Hexylisocyanat ab.

Entsprechend dem Destillatanfall wird der Kesselinnendruck während der Destillation vorsichtig weiter bis auf 20 mbar reduziert. Gegen Ende der Destillation wird das Rücklaufverhältnis auf volle Abnahme eingestellt.

Nach ca. 10 Stunden ist die Reaktion/Destillation beendet und das Sumpfprodukt wird nach dem Belüften mit Stickstoff auf 120°C abgekühlt. Bei dieser Temperatur ist der Sumpf noch gut fließfähig und kann durch Anlegen eines leichten Überdrucks ausgetragen und abgefüllt werden.

Ausbeute: 87 % d. Theorie
NCO-Gehalt: 33,1 %
Aussehen: klare, farblose Flüssigkeit
Anteil an n-Hexylisocyanat: 99,4 % (nach GC-Methode)

### Vergleichsbeispiel 2 ( nicht erfindungsgemäß, Laborversuch)

In einen 4000 ml Vierhalskolben mit Rührer, Innenthermometer und Destillationsbrücke werden 2100 g 4,4'-Diisocyanatodiphenylmethan (HC-Gehalt <10 ppm, bestimmt nach potentiometrischer Methode) eingefüllt, aufgeschmolzen und auf ca. 130°C aufgeheizt.

Bei dieser Temperatur werden 270 g n-Hexylamin zugetropft, wobei die Temperatur bis 160°C ansteigt.

Nach beendeter Zugabe wird vorsichtig evakuiert und das erhaltene Monoisocyanat abdestilliert. Die Reaktionstemperatur wird hierbei bis auf 190 bis 195°C erhöht. Nach 5 Stunden Reaktionszeit ist der Reaktionsansatz so hochviskos, dass der Vierhalskolben anschließend nicht mehr entleert werden kann. Trotz dieser drastischen Bedingungen bleibt mit 83 % die Ausbeute des abdestillierten Hexylisocyanats unter der des Produktionsversuchs (siehe Beispiel 1). Der Reaktionskolben ist nach Versuchsende nicht mehr zu reinigen und muss mit Inhalt verworfen werden.

## Patentansprüche

1. Verfahren zur Herstellung von niedrigsiedenden Monoisocyanaten mit einem Siedepunkt bei Normaldruck zwischen 70 und 320°C
A) aus einer hochsiedenden Isocyanatkomponente durch Umsetzung mit
B) einem Monoamin mit einer primären Aminogruppe in situ zu den entsprechenden Biuret/Harnstoffgruppen-haltigen Isocyanaten und deren gleichzeitiger Spaltung,
C) Abdestillieren des gebildeten Monoisocyanats gegebenenfalls unter Vakuum,
**dadurch gekennzeichnet, dass**
als hochsiedende Isocyanatkomponente Isocyanatverbindungen mit einem Siedepunkt bei Normaldruck von mindestens 180°C und einem Gehalt an hydrolisierbaren chlorverbindungen (HC-Gehalt) von mindestens 50 ppm eingesetzt werden, die Reaktion bei einer maximalen Reaktionstemperatur von 180°C in einem Verhältnis von Isocyanatgruppen zu Aminogruppen von mindestens 4 durchgeführt wird.

2. Verfahren nach Anspruch 1, **dadurch gekennzeichnet, dass** die hochsiedende Isocyanatkomponente A) einen HC-Gehalt von mindestens 300 ppm aufweist und die Reaktionstemperatur auf 160°C begrenzt ist.

3. Verfahren nach Anspruch 1 und 2 **dadurch gekennzeichnet, dass** als Monoamin B) ein aromatisches Monoamin eingesetzt wird.

## Claims

1. Process for the preparation of low-boiling monoisocyanates having a boiling point between 70 and 320°C at standard pressure
A) from a high-boiling isocyanate component by reaction with
B) a monoamine having a primary amino group, to form in situ the corresponding isocyanates which comprise biuret/urea groups, and the simultaneous breakdown thereof,
C) removal by distillation, optionally under vacuum, of the monoisocyanate formed,
**characterised in that**
isocyanate compounds having a boiling point of at least 180°C at standard pressure and a hydrolysable chlorine compounds content (HC content) of at least 50 ppm are used as the high-boiling isocyanate component, the reaction is carried out at a maximum reaction temperature of 180°C at a ratio of isocyanate groups to amino groups of at least 4.

2. Process according to Claim 1, **characterised in that** the high-boiling isocyanate component A) has an HC content of at least 300 ppm, and the reaction temperature is limited to 160°C.

3. Process according to Claims 1 and 2, **characterised in that** an aromatic monoamine is used as the monoamine B).

## Revendications

1. Procédé pour la préparation de monoisocyanates à bas point d'ébullition, c'est-à-dire à un point d'ébullition de 70 à 320°C à pression normale
A) à partir d'un composant isocyanate à haut point d'ébullition par réaction in situ avec
B) une monoamine à un groupe amino primaire, la réaction donnant les isocyanates correspondants à groupes biuret/urée qui sont simultanément scindés,
C) distillation du monoisocyanate formé, éventuellement sous vide,
**caractérisé en ce que**
on met en oeuvre, en tant que composants isocyanates à haut point d'ébullition, des isocyanates ayant un point d'ébullition d'au moins 180°C à pression normale et à une teneur en composés chlorés hydrolysables d'au moins 50 ppm, on exécute la réaction à une température maximale de 180°C, avec un rapport d'au moins 4 entre les groupes isocyanates et les groupes amino.

2. Procédé selon la revendication 1, **caractérisé en ce que** le composant isocyanate à haut point d'ébullition A) a une teneur en composés chlorés hydrolysables d'au moins 300 ppm et **en ce que** la température de réaction est limitée à 160°C.

3. Procédé selon les revendications 1 et 2, **caractérisé en ce que** l'on utilise en tant que monoamine B) une monoamine aromatique.
